# EUROPEAN PATENT SPECIFICATION

(11) **EP 3 269 351 B1**
(45) Date of publication and mention of the grant of the patent: **18.11.2020**
(21) Application number: 16765182.7
(22) Date of filing: 08.03.2016
(51) Int. Cl.: C01G 23/08, A61K 8/29, A61Q 1/00, A61K 8/81

(54) **COMPOSITE POWDER HAVING SURFACE OF INORGANIC POWDER COVERED WITH PRESSURE-SENSITIVE ADHESIVE POLYMER, COSMETIC COMPOSITION CONTAINING SAME, AND METHOD FOR PREPARING SAME**
VERBUNDPULVER MIT OBERFLÄCHE AUS ANORGANISCHEM PULVER, DIE MIT DRUCKEMPFINDLICHEM KLEBENDEM POLYMER BEDECKT IST, KOSMETISCHE ZUSAMMENSETZUNG DAMIT UND VERFAHREN ZUR HERSTELLUNG DAVON
POUDRE COMPOSITE DONT LA SURFACE EN POUDRE INORGANIQUE EST RECOUVERTE D'UN POLYMÈRE ADHÉSIF DÉCOMPRIMÉ, COMPOSITION COSMÉTIQUE LA CONTENANT ET PROCÉDÉ POUR LA PRÉPARER

(30) Priority: 13.03.2015 KR 20150035098
(43) Date of publication of application: 17.01.2018
(73) Proprietor: Amorepacific Corporation, Seoul 04542 (KR)
(72) Inventor: LEE, Hyun Suk, Yongin-si Gyeonggi-do 17074 (KR); KIM, Yong Jin, Yongin-si Gyeonggi-do 17074 (KR); LEE, Jon Hwan, Yongin-si Gyeonggi-do 17074 (KR); LEE, Su Jin, Yongin-si Gyeonggi-do 17074 (KR); CHOI, Kyung Ho, Yongin-si Gyeonggi-do 17074 (KR); CHOI, Yeong Jin, Yongin-si Gyeonggi-do 17074 (KR); PARK, Seung Han, Yongin-si Gyeonggi-do 17074 (KR); CHOI, Sung-Wook, Bucheon-si Gyeonggi-do 14613 (KR); JUN, Dae-Ryong, Seoul 07043 (KR)
(74) Representative: dompatent von Kreisler Selting Werner - Partnerschaft von Patent- und Rechtsanwälten mbB
(86) International application number: PCT/KR2016/002302
(87) International publication number: WO 2016/148426

(56) References cited:
- JP-B2- 3 959 205
- JP-B2- 3 959 205
- KR-B1- 100 795 233
- US-A- 5 876 855
- US-A1- 2005 186 165
- US-A1- 2008 159 965
- US-A1- 2009 317 451
- TURNER D T ET AL: "The glass transition temperature of poly(N-vinyl pyrrolidone) by differential scanning calorimetry", POLYMER, ELSEVIER SCIENCE PUBLISHERS B.V, GB, vol. 26, no. 5, 1 May 1985 (1985-05-01), pages 757-762, XP024115026, ISSN: 0032-3861, DOI: 10.1016/0032-3861(85)90114-4 [retrieved on 1985-05-01]
- Robert A Shankst ET AL: "Effect o| Monomer Composition on the Adhesion ot Pressure-sensitive Adhesives Prepared by Photoinitiated Polymerization", Polymer Photochemistry, vol. 4, 1 January 1984 (1984-01-01), pages 451-458, XP055562177,

## Description

### [Technical Field]

The present invention relates to a composite powder containing an inorganic powder whose surface is coated with a pressure-sensitive adhesive (PSA) polymer, and more particularly, to a composite powder having improved skin adhesiveness because a surface of an inorganic powder contained in cosmetics is uniformly coated with a pressure-sensitive adhesive polymer, a cosmetic composition containing the same, and a method for preparing the same.

### [Background Art]

Makeups play an important role in correction of skin blemishes and emphasis of eyes and lips, making the appearance look beautiful. Among the components mixed in makeup cosmetics, coloring materials are used to conceal stains or marks, allowing the skin to look healthy and be tinted with fascinating tones. The coloring materials used in these cosmetics are divided into organic synthetic coloring agents, natural colors, and inorganic pigments.

The organic synthetic coloring agents are classified into a dye, lake, and an organic pigment. The dye is a compound which is dissolved in water, oil, and alcohol, and contains an azo group and the like as a chromophore group, and the lake is a type of an insoluble metal salt bound to a water-soluble dye. The natural colors are extracted from plants, animals, and microorganisms. In this case, such materials have been used as additive materials in foods for a long time because the materials produce poor colors, compared to the synthetic coloring agents. In recent years, the natural colors have again come into the spotlight since the natural colors are used for medicines requiring the safety.

The inorganic pigments are classified into coloring pigments, white pigments, extender pigments, and pearlescent pigments. The coloring pigments give colors to cosmetics, and the white pigments serve to regulate skin coverage power. The extender pigments serve to adjust tones as diluents for coloring pigments and play a great role in senses of feeling in use, such as spreadability, adhesiveness, etc., of products, and formulation of the products. Also, the pearlescent pigments are pigments that endow colors with pearly luster and have a special optical effect, which are used to give an iris color or a metallic luster.

The extender pigments included in conventional cosmetics include talc, kaolin, silica, talc, sericite, calcium carbonate, magnesium carbonate, a silicic acid anhydride, etc., and serve to adjust the depth of color and control spreadability and feeling of formulations, etc. The white pigments include titanium dioxide, zinc oxide, etc. In general, basic components of the cosmetics include kaolin, talc, silica, and titanium dioxide as powders having white color while maintaining applicability, adhesion and skin coverage power.

The powders used in the makeup cosmetics require adhesion, hiding power, applicability, spreadability, moisture absorbency, oil absorbency, and skin friendliness, and may be obtained by mixing or applying two or more powders. For example, the inorganic pigments such as titanium dioxide exhibit excellent adhesion, hiding power, etc., but have poor senses of feeling in use such as applicability, spreadability, etc. To make up for these drawbacks, there are known techniques for preparing cosmetics using good hiding power and difference in optical refractive index by coating titanium dioxide with silica at a predetermined ratio to prepare an inorganic composite powder.

However, the conventional coated inorganic composite powder as described above has a problem in that it do not exhibit satisfactory characteristics such as stability, adhesion, a sense of feeling in use, and sustainability under the skin and external conditions when actually applied onto the skin.

As a conventional plan to solve these drawbacks, methods of performing water repellency treatment using surface treating agents such as silicon compounds, fluorine compounds, metallic soaps, higher alcohols, fatty acids, metal salts of amino acids, lecithin, and the like have been proposed in the related art. Among theses, a surface treatment method using silicon compounds or metal salts of amino acids is common. In recent years, a surface treatment method using a combination of the two methods has been proposed.

Meanwhile, the coated powder whose surface is treated with a fatty acid, a metallic soap, and a metal salt has good adhesion to the skin and a good moist sense of feeling in use on the skin, but has poorer water resistance than the powder surface-treated with the silicon compound and exhibits insufficient spreadability due to somewhat a rough sense of feeling when applied onto the skin. Also, because the metal salt is absorbed onto a surface of the powder in the form of fine particles, the metal salt may be detached due to poor stability in a surface-modified state. In addition, the coated powder still has problems regarding degraded color reproduction when used together with other silicon raw materials.

JP 3 959205 B2 discloses a powder characterized in that its surface is treated with polyvinylpyrrolidone. Shanks, R.A. and Clarke, S.R., 1984. Effect of monomer composition on the adhesion of pressure-sensitive adhesives prepared by photoinitiated polymerization. Polymer photochemistry, 4(6), pp.451-458, discloses a method for preparing pressure-sensitive adhesives directly on the backing substrate by photoinitiated polymerization.

### [Prior-art Document]

Registered Korean Patent No. 0795233: "Makeup Cosmetic Composition Including Composite Pigment Having Improved Water/Oil Repellence and Coating Uniformity and Method of Preparing the Same"

### [Disclosure]

### [Technical Problem]

In general, makeup cosmetics are applied to the outermost layer of facial skin when someone wants to put on the makeup, and thus require skin adhesiveness at a level higher than a predetermined level. As a method of improving adhesion, a method of increasing a content of wax having high hardness among oily components, such as ozokerite, carnauba wax, beeswax, etc., or a method of increasing a content of an organic polymer serving as a thickening agent, an inorganic thickening agent or an organic/inorganic hybrid thickening agent has been known. However, because an increase in content of the wax or thickening agent among the oily components results in increased density of a formulation forming crystals among the oily component with respect to the total volume of makeup cosmetics, a drop in fluidity may be caused, causing degradation of applicability and spreadability when the makeup cosmetics are applied to the skin. Therefore, the makeup cosmetics have a drawback in that they have a poor ease of use and a sense of thick feeling in use.

### [Technical Solution]

To solve the above problems, the present invention provides a composite powder containing an inorganic powder, i.e. titanium dioxide powder, whose surface is coated with a pressure-sensitive adhesive polymer, i.e. a copolymer of N-vinyl pyrrolidone and butyl acrylate, a method for preparing the same, and a cosmetic composition including the same. In this case, the pressure-sensitive adhesive polymer may be a combination of two or more components, and is used by adjusting a difference in glass transition temperature (Tg) and a content ratio.

The embodiments of the present invention are reflected in independent claims 1 to 3.

### [Advantageous Effects]

The makeup cosmetics of the present invention, which includes the composite powder containing the inorganic powder whose surface is coated with the pressure-sensitive adhesive polymer, has adhesiveness resulting from the pressure-sensitive adhesive polymer when pressed with an applying and absorbing means (for example, a hand or a puff), thereby exhibiting excellent skin adhesiveness, and thus improved coverage and lasting power.

Also, the makeup cosmetics of the present invention, which includes the composite powder containing the inorganic powder whose surface is coated with the pressure-sensitive adhesive polymer, has excellent oil dispersibility and superior applicability when applied on the skin, and has an effect of bringing a light and refreshing sense of feeling in use.

### [Description of Drawings]

FIG. 1 is a schematic explanatory view of a method for preparing a composite powder according to the present invention.
FIG. 2 is an enlarged image of a surface of a composite powder containing an inorganic powder whose surface is coated with a pressure-sensitive adhesive polymer according to the present invention.
FIG. 3 is an image obtained by performing an adhesiveness test on the composite powder containing an inorganic powder whose surface is coated with a pressure-sensitive adhesive polymer according to the present invention, the adhesiveness test being performed using a durometer.
FIG. 4 is a diagram showing results of the adhesiveness test on the composite powder containing an inorganic powder whose surface is coated with a pressure-sensitive adhesive polymer according to the present invention.
FIG. 5 is an image obtained by performing a viscoelasticity test on the composite powder containing an inorganic powder whose surface is coated with a pressure-sensitive adhesive polymer according to the present invention, the viscoelasticity test being performed using a rheometer.
FIG. 6 is a diagram showing results of the viscoelasticity test on the composite powder containing an inorganic powder whose surface is coated with a pressure-sensitive adhesive polymer according to the present invention.

### [Best Mode]

The present invention relates to a composite powder containing an inorganic powder whose surface is coated with a pressure-sensitive adhesive (PSA) polymer, and more particularly, to a composite powder having improved skin adhesiveness because a surface of an inorganic powder contained in the cosmetics is uniformly coated with a pressure-sensitive adhesive polymer, a cosmetic composition containing the same, and a method for preparing the same.

Hereinafter, the present invention will be described in further detail with reference to the specific embodiments thereof. However, it will be apparent to a person having ordinary skill in the art that the present invention is not limited to the following embodiments, and various modifications and changes can be made without departing from the scope of the invention. Unless otherwise defined in this specification, all the technical and scientific terms used herein also have the same meanings as what are generally understood by a person skilled in the related art to which the present invention belongs. Therefore, repeated descriptions of the same technical configurations and actions as in the prior art are omitted for clarity.

In this specification, the term "pressure-sensitive adhesive (PSA) polymer" refers to a polymer that exhibits adhesiveness as a pressure-sensitive polymer when a pressure is applied to attach it to an adhesive surface. Based on this principle, the present inventors have tried to enable cosmetics to exhibit skin adhesiveness due to the adhesiveness resulting from a pressure-sensitive adhesive (PSA) polymer when pressed during application and absorption of the makeup cosmetics, and found this technique of coating a surface of an inorganic powder applied to the makeup cosmetics with a pressure-sensitive adhesive polymer. Therefore, the present invention has been completed based on these facts.

In the present disclosure the pressure-sensitive adhesive polymer with which the surface of the inorganic powder is coated may include one selected from the group consisting of polyvinyl pyrrolidone (PVP), polymethyl methacrylate (PMMA), polystyrene (PS), polyaspartic acid (PAA), poly-2-hydroxy ethyl methacrylate (P2HEMA), polystearyl methacrylate (PSMA), poly-2-hydroxy propyl methacrylate (P2HPMA), poly-n-butyl methacrylate (PBMA), polyethyl acrylate (PEA), polybutyl acrylate (PBA), poly-2-ethyl hexyl acrylate (P2EHA), and a combination thereof,

The inorganic powder is titanium dioxide,

A combination of two or more pressure-sensitive adhesive polymers may be used as the composite powder according to the present invention, when necessary. When the pressure-sensitive adhesive polymer has a low glass transition temperature (Tg), initial adhesiveness may be improved due to increased plasticity, resulting in enhanced adhesion. On the other hand, when the pressure-sensitive adhesive polymer has a high glass transition temperature (Tg), applicability and a sense of feeling in use may be improved due to high fluidity. Therefore, when a combination of pressure-sensitive adhesive polymers having different glass transition temperatures is used, it is possible to obtain an effect of mutually complementing the adhesion caused due to the adhesiveness and the applicability and sense of feeling in use caused due to the fluidity.

The following Table 1 lists glass transition temperatures (Tg) of pressure-sensitive adhesive polymers applicable to coating a surface of an inorganic powder according to the present invention.

**[Table 1]**

| PSA polymers | Glass transition temperature (Tg) |
|---|---|
| Polyvinyl pyrrolidone (PVP) | 150 °C |
| Polymethyl methacrylate (PMMA) | 105 °C |
| Polystyrene (PS) | 95 °C |
| Polyaspartic acid (PAA) | 87 °C |
| Poly-2-hydroxy ethyl methacrylate (P2HEMA) | 55 °C |
| Polystearyl methacrylate (PSMA) | 38 °C |
| Poly-2-hydroxy propyl methacrylate (P2HPMA) | 26 °C |
| Poly-n-butyl methacrylate (PBMA) | 15 °C |
| Polyethyl acrylate (PEA) | -23 °C |
| Polybutyl acrylate (PBA) | -50 °C |
| Poly-2-ethyl hexyl acrylate (P2EHA) | -70 °C |

Among the pressure-sensitive adhesive polymers listed in Table 1, a combination of two polymers whose glass transition temperatures (Tg) differ by 100 °C or higher is preferably used. According to one exemplary embodiment of the present disclosure the combinations of two polymers are prepared to include a combination (PBMA + PVP) of poly-n-butyl methacrylate (PBMA) and polyvinyl pyrrolidone (PVP), a combination (PBA + PMMA) of polybutyl acrylate (PBA) and polymethyl methacrylate (PMMA), and a combination (PBA + PVP) of polybutyl acrylate (PBA) and polyvinyl pyrrolidone (PVP).

To uniformly coat a surface of the inorganic powder with the pressure-sensitive adhesive polymer, the composite powder of the present invention is preferably prepared through dispersion polymerization. Specifically, the composite powder of the present disclosure may be prepared by a method including:
1) dispersing an inorganic powder in a solvent to prepare a solution;
2) adding hydrochloric acid and methacrylic acid (MAA) to the solution of step 1) to form an inorganic powder having a positive (+) dipole moment; and
3) adding a monomer for the pressure-sensitive adhesive polymer and a polymerization initiator to the solution of step 2) to polymerize a polymer on a surface of the inorganic powder so that the surface of the inorganic powder is coated with the polymer.

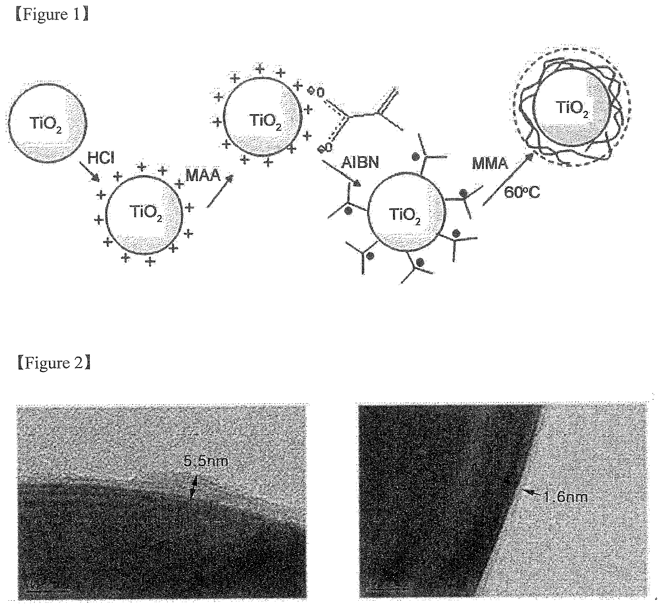

FIG. 1 is an explanatory view for schematically explaining the method for the cases in which titanium dioxide, methacrylic acid (MAA), and azobisisobutyronitrile (AIBN) are used as the inorganic powder, the monomer for the pressure-sensitive adhesive polymer, and the polymerization initiator.

According to one exemplary embodiment of the present invention, a formulation of the cosmetics, which include the composite powder containing the inorganic powder whose surface is coated with the pressure-sensitive adhesive polymer, is prepared into liquid foundations. For example, formulations may be suitably chosen according to a purpose, but the present invention is not particularly limited thereto. The cosmetic composition of the present invention may be formulated into, for example, powders, powder pacts, two-way cakes, powder foundations, liquid foundations, cream foundations, concealers, BB creams, CC creams, makeup bases, primers, eyeshadows, blushers, etc.

In addition to the composite powder containing the inorganic powder whose surface is coated with the pressure-sensitive adhesive polymer, the cosmetic composition of the present invention may further include a functional additive and components included in the conventional cosmetic compositions. The functional additive may include components selected from the group consisting of water-soluble vitamins, oil-soluble vitamins, polymeric peptides, polymeric polysaccharides, sphingolipids, and seaweed extracts. Also, the components included in the conventional cosmetic composition may be mixed together with the functional additive, when necessary. In addition, blending components included in the cosmetic composition may include an oily component, a moisturizing agent, an emollient, a surfactant, organic and inorganic pigments, an organic powder, a UV absorbent, a preservative, a bactericide, an antioxidant, a plant extract, a pH regulator, an alcohol, a color, a fragrance, a blood flow stimulant, a cooling agent, a control agent, purified water, etc.

### [Mode for Invention]

Hereinafter, preferred embodiments of the present invention are provided to aid in understanding the present invention. However, it will be apparent to a person having ordinary skill in the art that the present invention is not limited to the preferred embodiments, and other applications and modifications are made to the present invention.

### Example 1: Preparation of composite powder

Ten composite powders were prepared through dispersion polymerization to have configurations listed in the following Table 2 with the varying types and contents of the monomers for the pressure-sensitive adhesive polymer. Composite powder P8 represents a working example according to the present invention. Other composite powders are comparative examples

**[Table 2]**

| | Inorganic powder | Pressure-sensitive adhesive polymer | |
|---|---|---|---|
| P1 | TiO₂ | 3% PMMA | |
| P2 | TiO₂ | 3% PBMA | |
| P3 | TiO₂ | 3% PSMA | |
| P4 | TiO₂ | 3% PVP | |
| P5 | TiO₂ | 3% PMAA | |
| P6 | TiO₂ | 4% PMMA | 1% PVP |
| P7 | TiO₂ | 4% PBMA | 1% PVP |
| P8 | TiO₂ | 2% PBA | 2% PVP |
| P9 | TiO₂ | 2% PBA | 1% PMMA |
| P10 | TiO₂ | 2% PBA | 2% PMMA |

Among the configurations listed in Table 2, a process of preparing a composite powder P8 was as follows, and the composite powders other than P8 were also obtained in the same manner, except that different monomers were used, respectively, in step 5).
1) A double jacket reactor was installed, and a circulating water bath was connected using an overhead stirrer. Thereafter, the temperature was set to 70 °C, cooling water was allowed to flow in a condenser, and a septum was mounted to shut off the air from the outside.
2) 157 g of methanol and 67 g of distilled water were added to the reactor to prepare a dispersion.
3) 24 g of titanium dioxide (TiO₂; CR-50 commercially available from Sunjin Chemical Co., Ltd.) was dispersed in 100 g of methanol and 100 µL of HCl for 5 minutes using ultrasonic waves (5 seconds on/5seconds off), and the resulting dispersion was then kept in an iced water bath to prevent an increase in temperature.
4) The titanium dioxide dispersion prepared in step 3) was added to the reactor.
5) As a polymerization initiator, 0.024 g of azobisisobutyronitrile (AIBN) was added to monomers of 1.2 g of N-vinyl pyrrolidone and 1.2 g of butyl acrylate (BA), and then stirred until the AIBN was completely dissolved.
6) When the reactor was heated to a temperature of 70 °C, the monomer mixture of step 5) was added dropwise using a syringe to prevent the mixture from coming in contact with the walls of the reactor.
7) The resulting mixture was reacted for 6 hours, and the reaction product was then recovered.

FIG. 2 is an enlarged image of a surface of the composite powder (P8) of titanium dioxide (TiO₂) whose surface is coated with the PVP and PBA while the PVP and PBA are polymerized at a weight ratio of 1:1. It was revealed that the pressure-sensitive adhesive polymers, PVP and PBA, were formed to have a thickness spanning from a minimum of 1.6 nm to a maximum of 5.5 nm.

### Example 2: Preparation of cosmetics containing composite powder

The foundations containing the composite powders P1 to P10 listed in Table 2 were prepared using the same components.

### Comparative Example 1: Preparation of cosmetics containing simple titanium dioxide inorganic powder

P11 to P13 listed in the following Table 3 included titanium dioxide inorganic powders contained in the conventional foundations. Foundations containing P11 to P13 were prepared using the same components as in Example 2. In this case, OTS represents n-octyl triethoxy silane.

**[Table 3]**

| | Inorganic powder | Polymer | Product name |
|---|---|---|---|
| P11 | TiO₂ | 2% OTS | OTS-2 TiO₂ CR50 |
| P12 | TiO₂ | 2% lecithin | LTS-TiO₂ R250 |
| P13 | TiO₂ | 5% disodium stearoyl glutamate | NAI PFC-407 |

### Test Example 1: Adhesion test

As a comparison table, the following Table 4 lists the results obtained by performing an adhesion test on the 13 foundation thus prepared. Adhesion evaluations were performed by an expert panel of 10 examiners, and an evaluation method was performed by drawing cells on the fore arm at a size of 0.4 × 1.5 cm and counting the rolling number until 40 µL of contents were completely adhered to the fore arm.

**[Table 4]**

| | Inorganic powder | Polymer | | Adhesion evaluation (Grade 10) | Feeling characteristics in use |
|---|---|---|---|---|---|
| P1 | TiO₂ | 3% PMMA | | 4 points | Feeling thick/residual |
| P2 | TiO₂ | 3% PBMA | | 7 points | |
| P3 | TiO₂ | 3% PSMA | | 8 points | |
| P4 | TiO₂ | 3% PVP | | 7 points | Slightly adhered |
| P5 | TiO₂ | 3% PMMA | | 5 points | |
| P6 | TiO₂ | 4% PMMA | 1% PVP | 7 points | Feeling weighty |
| P7 | TiO₂ | 4% PBMA | 1% PVP | 4 points | |
| P8 | TiO₂ | 2% PBA | 2% PVP | 9 points | Slightly adhered |
| P9 | TiO₂ | 3% PBA | 1% PMMA | 5 points | Feeling thick |
| P10 | TiO₂ | 2% PBA | 2% PMMA | 5 points | |
| P11 | TiO₂ | OTS 2% | | 2 points | Slightly adhered/no feeling residual/insufficient adhesion |
| P12 | TiO₂ | 2% lecithin | | 4 points | |
| P13 | TiO₂ | 5% disodium stearoyl glutamate | | 3 points | |

From the results of Table 4, it was revealed that the foundation P8 containing titanium dioxide whose surface was coated with the pressure-sensitive adhesive polymer prepared in the preparative example of the present invention were generally evaluated to have superior adhesion, compared to the conventional foundations P11 to P13 and the comparative foundations P1 to P7 and P9 to P10.

Also, a questionnaire survey including a residual touch item was performed on the expert panel after the adhesion evaluation. As a result, the formulation (P3) including PSMA was evaluated to have the highest feeling of residual touch, and the formulations (PI and P2) including PBMA and PMMA were evaluated to have the next highest feeling of residual touch. Therefore, when the formulations were used alone, the formulations were unsuitable for prescription of products. On the other hand, the formulation (P4) including PVP was evaluated to feel light and refreshing. As a result, the formulation giving a light sense of feeling and having the highest adhesion was evaluated to be the formulation (P8) including PBA and PVP at a mass ratio of 1:1.

### Test Example 2: Oil dispersibility test

A degree of stability was measured for a week in a temperature-controllable shaking incubator. One temperature cycle was as follows: Measurements were performed over a period of 8 hours at each of temperatures of -10 °C, 30 °C, and 45 °C, and performed for one cycle a day for 7 days to determine whether problems such as oil separation, emulsion separation, and the like occurred. The data were obtained a total of 5 times from the formulations including P1 to P13. As a result, it was revealed that all the formulations had good oil dispersibility.

### Test Example 3: Adhesiveness test

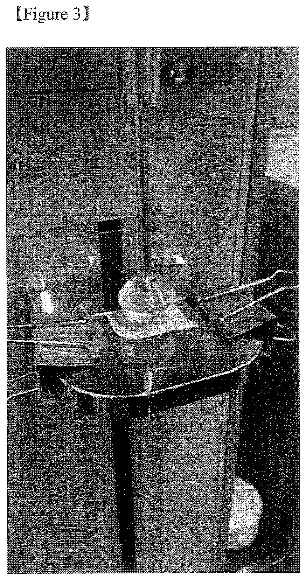

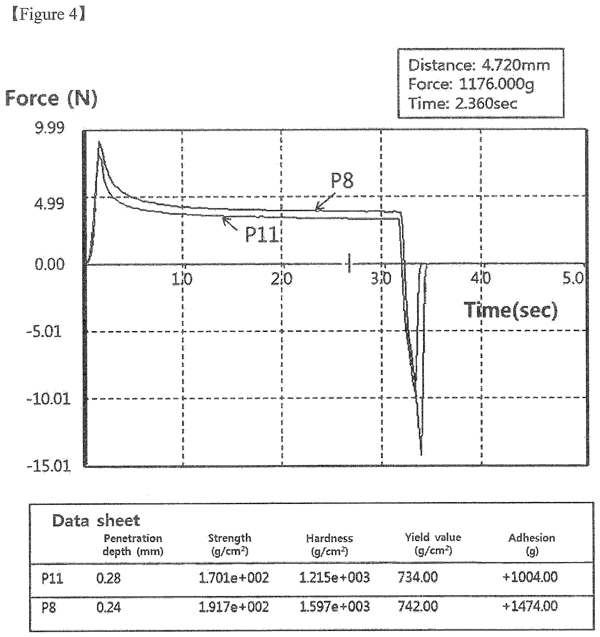

For a more objective test, an adhesiveness test was performed. FIGS. 3 and 4 are an image obtained by performing an adhesiveness test using a durometer, and a diagram showing the results of the adhesiveness test. The tests were performed on the formulation containing the conventional P11 powder and the formulation containing the P8 composite powder of the present invention under conditions of a distance of 4.720 mm, a force of 1,176.000 g and a time of 2.360 seconds. The test results showed that the adhesiveness of the formulation containing the P8 composite powder of the present invention increased by approximately 5 N, compared to that of the formulation containing the conventional P11 powder.

### Test Example 4: Viscoelasticity test

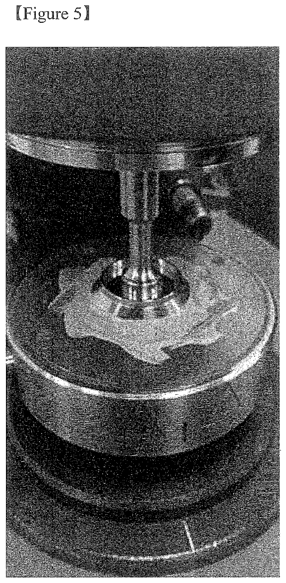

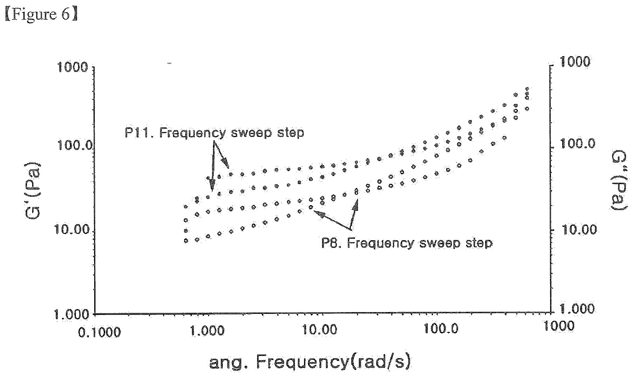

FIGS. 5 and 6 are an image obtained by performing a viscoelasticity test using a rheometer, and a diagram showing the results of the viscoelasticity test. The tests were performed on the formulation containing the conventional P11 powder and the formulation containing the P8 composite powder of the present invention. As a result, it was revealed that the formulation containing the P8 composite powder of the present invention had more flowability than the formulation containing the conventional P11 powder because the storage modulus (G°) of the formulation containing the P8 composite powder of the present invention was measured to be lower than that of the formulation containing the conventional P11 powder. Therefore, the formulation containing the P8 composite powder of the present invention was evaluated to have superior applicability when the formulation was applied to the skin.

From the results of Test Examples 1 to 4, it can be seen that the cosmetics formulation of the present invention, which included the composite powder containing titanium dioxide whose surface was coated with PBA and PVP at a mass ratio of 1:1, had superior adhesion and applicability, compared to the cosmetics formulation including the conventional simple titanium dioxide inorganic powder.

## Claims

1. A composite powder containing titanium dioxide powder whose surface is coated with a copolymer of N-vinyl pyrrolidone and butyl acrylate wherein the N-vinyl pyrrolidone and butyl acrylate are at a weight ratio of 1:1.

2. A cosmetic composition including the composite powder defined in claim 1.

3. A method for preparing a composite powder according to claim 1, comprising:
1) dispersing a titanium dioxide powder in a solvent to prepare a solution;
2) adding hydrochloric acid to the solution of step 1) to form titanium dioxide powder having a positive (+) dipole moment; and
3) adding N-vinyl pyrrolidone, butyl acrylate and a polymerization initiator to the solution of step 2) to coat a copolymer on a surface of the inorganic powder.

## Patentansprüche

1. Verbundpulver, das Titandioxidpulver enthält, dessen Oberfläche mit einem Copolymer von N-Vinylpyrrolidon und Butylacrylat überzogen ist, wobei das N-Vinylpyrrolidon und das Butylacrylat in einem Gewichtsverhältnis von 1:1 vorliegen.

2. Kosmetikzusammensetzung, die das Verbundpulver gemäß Anspruch 1 umfasst.

3. Verfahren zur Herstellung eines Verbundpulvers gemäß Anspruch 1, umfassend:
1) Dispergieren eines Titandioxidpulvers in einem Lösungsmittel zur Herstellung einer Lösung;
2) Hinzufügen von Chlorwasserstoffsäure zu der Lösung von Schritt 1) unter Bildung von Titandioxidpulver, das ein positives (+) Dipolmoment aufweist; und
3) Hinzufügen von N-Vinylpyrrolidon, Butylacrylat und eines Polymerisationsstarters zu der Lösung von Schritt 2), um die Oberfläche des anorganischen Pulvers mit einem Copolymer zu überziehen.

## Revendications

1. Poudre composite contenant une poudre de dioxyde de titane la surface de laquelle est enrobée avec un copolymère de N-vinylpyrrolidone et d'acrylate de butyle, dans laquelle ladite N-vinylpyrrolidone et ledit acrylate de butyle sont présents dans un rapport pondéral de 1 : 1.

2. Composition cosmétique, comprenant la poudre composite selon la revendication 1.

3. Procédé pour préparer une poudre composite selon la revendication 1, comprenant les étapes consistant à :
1) disperser une poudre de dioxyde de titane dans un solvant pour préparer une solution,
2) ajouter de l'acide chlorhydrique à la solution provenant de l'étape 1) pour former une poudre de dioxyde de titane ayant un moment dipolaire positif (+), et
3) ajouter de la N-vinylpyrrolidone, de l'acrylate de butyle et un initiateur de polymérisation à la solution provenant de l'étape 2) pour enrober une surface de la poudre inorganique avec un copolymère.
